# EUROPEAN PATENT SPECIFICATION

(11) **EP 2 649 986 B1**
(45) Date of publication and mention of the grant of the patent: **25.07.2018**
(21) Application number: 13156228.2
(22) Date of filing: 21.02.2013
(51) Int. Cl.: A61K 8/67, A61K 8/49, A61Q 19/08

(54) **Cosmetic composition for rejuvenating the skin**
Kosmetische Zusammensetzung zur Verjüngung der Haut
Composition cosmétique de rajeunissement de la peau

(30) Priority: 22.02.2012 ES 201230265
(43) Date of publication of application: 16.10.2013
(73) Proprietor: Umbert Millet, Ignacio, 08017 Barcelona (ES)
(72) Inventor: Umbert Millet, Ignacio, 08017 Barcelona (ES)
(74) Representative: Curell Suñol S.L.P.

(56) References cited:
- WO-A1-2009/037093
- WO-A2-98/55075

## Description

### Technical sector of the invention

This invention relates to a cosmetic composition for rejuvenating the skin ("antiaging") containing retinaldehyde or, generally, a retinoic acid precursor as listed in claim 1. The invention also relates to a method for rejuvenating the skin, and to some uses of the compositions according to the invention.

### Background of the invention

For several years now, retinoic acid, or acidic forms of vitamin A, has been used to treat a variety of skin affections, for example, aging, acne, wrinkles, psoriasis, age blemishes and discolouring. See for example, 1) Vahlquist, A. and col., J. Invest. zermatol., Vol. 94, Holland D. B. and Cunliffe, W. J. (1990), pag. 496-498; Ellis, C. N. and col., "Pharmacology of Retinols in Skin", Vasel, Karger, Vol. 3, (1989), pag. 249-252; Lowe, N. J. and col., "Pharmacology of Retinols in Skin", Vol. 3, (1989), pag. 240-248; 2) Fourie, Stephanus Petrus 'Dermatological preparation'. CHEMICAL ABSTRACTS, vol. 90, no. 12, 19 March 1979, Columbus, Ohio, US; abstract no. 92433; 3) Kligman, AM, "Guidelines for the use of topical tretinoin (Retin-A) for photoaged skin", J Am. Acad. Dermatol. 1989; 21:650-4; 4) Kligman and col., "Topical tretionoin for photoaged skin", J Am. Acad. Dermatol. 1986; 15:836-59.

The documents of patents US 3.856.934, 2. WO2009/037093, WO98/55075 WO 93/19743, US 5.998.395, GB-A 906.000, FR 2.785.185 and US 6.319.957 describe dermatological or cosmetic uses of retinoic acid forms. The latter two describe formulae that contain retinoic acid and US 6.319.957 describes a cosmetic formula that also includes an anti-inflammatory substance.

Document US 6.319.957 defines a composition comprising retinoic acid, a corticoid, and hydroquinone. As hydroquinone is a substance that is being contraindicated or even prohibited in some countries, hydroquinone must be replaced with a technical equivalent thereof, insofar as its anti-pigmenting function is concerned.

The above formulae are not without their drawbacks, consisting mainly in the fact that their therapeutic effect is very variable and unpredictable. According to these inventors, the variability is to a large degree due to the metabolic differences of the patients treated.

It is known that oxidative stress caused by the reactive oxygen species (ROS) that are produced in the skin, causes aggravated ageing and undesirable atypical pigmentation. Also, the reactive oxygen species induce specific cell signalling cascades that will have disastrous effects on the tissue structure measured by the development of an important inflammatory response.

Therefore, to block the undesired effects of oxidative stress, it is important to block both the ROS that are produced and also the associated inflammatory response. All with the aim of stopping the tissue deterioration and improving the skin structure.

With this objective, patent document US 20110262373 describes a treatment that effectively blocks both processes by using retinoic acid combined with different anti-oxidants. This combination not only counteracts the ROS produced but also will block the inflammatory response. Patent US 20110262373 describes an antiaging medicine that is based on retinoic acid with medical effects, associated synergistically with at least an anti-inflammatory (indometacin), anti-oxidants and vitamins, with or without hyaluronic acid.

Patent US 20110262373 provides a formula of a medical substance, and not simply cosmetic, that provides a solution to the above-explained problem, and is equally effective irrespective of the particular characteristics of the subject patients Despite the synergy of the components, which allows lowering the toxicity to low levels, this medicine is not completely free of irritative effects, because in short it is a medical substance.

It has been considered that it would be interesting to be able to lower the toxicity or aggression to cosmetic and not medical levels, using a percusor of retinoic acid, like retinaldehyde or other percusors, used in cosmetic proportions, but this has not been put into practice, to date, since there are two obstacles:
- the potential step of retinaldehyde to retinoic acid, which can lead to the same drawbacks already highlighted, and
- that the therapeutic effect is very reduced with respect to US 20110262373.

Patent EP 1.689.356 by Pierre Fabre Dermo-Cosmétique, describes compositions aimed at the topical application, comprising as active ingredient fragments of sodium hyaluronate (with a molecular weight between 50,000 and 750,000 Da) and retinal, but it is not clear that this provides a solution to the above-mentioned problems. Also, it is possible that it enhances them. But the hyaluronate is a salt and not an acid, although one can be obtained from the other. It does not describe whether hyaluronic acid is included in the combination. Also on the market cosmetic anti-wrinkle or antiaging creams are known by the same previous owner, which contain a mixture of retinaldehyde and hyaluronic acid.

However neither patent EP 1.689.356 or the cosmetic anti-wrinkle or antiaging creams indicated above have the effectiveness of the retinoic acid based medicine, q.v. that described in US 20110262373. The drawback of the medicines with respect to the cosmetic products is their higher potential of producing a toxic effect, normally due to the greater proportion of medical active ingredients.

The aim of this invention is a cosmetic antiaging product that has the effectiveness of a pharmaceutical product, but which is exempt of its irritative or toxic effects.

### Explanation of the invention

For this, the inventors have created a new cream which essentially is characterized by variable proportions of between 0.01 % and 0.5% by weight with respect to the total weight of the composition of a retinoic acid precursor, as defined in the appended claims, and between 0.05 % and 5% by weight with respect to the total weight of the composition of melatonin.

Preferably, the retinoic acid precursor is retinaldehyde. Alternatively, it can be retinol or retinyl esters, e.g. retinyl retinoate, in a proportion between 0.01 and 0.5% by weight with respect to the total weight of the composition. In this description and claims all the % are % by weight with respect to the total weight of the composition.

Preferably, the proportion of retinaldehyde is between 0.05% and 0.1% by weight with respect to the total weight of the composition.

The melatonin is included preferably in a proportion between 0.05 and 5% by weight with respect to the total weight of the composition, and preferably between 0.5 and 1%.

In the preferred embodiment, the composition includes also at least one anti-inflammatory other than melatonin, an anti-oxidant other than melatonin and/or a vitamin. Preferably the anti-inflammatory other than melatonin is an anti-inflammatory from the group made up of liquorice extract, aloe vera, alpha-bisabolol, azulene, glycyrrhetic acid and rosmarinum oficinalis extract and, more preferably, it is from the group made up of aloe vera and liquorice extract.

In an advantageous cosmetic formula it is proposed to associate 3 activities possessed by three groups of substances which together with the retinaldehyde obtain an antiaging action of this substance higher than that obtained on its own or with other groups of substances with another activity.

The cosmetic formula is made up of retinaldehyde as the essential substance, always in association with melatonin, an anti-oxidant that enhances the action of retinaldehyde, and preferably from the following groups of substances which, given the synergistic effect of this activity, enhance the antiaging action of retinaldehyde:
1st Group: substance with antiaging action: retinaldehyde (aldehydic form of retinol used in cosmetics) + melatonin
2nd Group: substances with anti-inflammatory action authorized for use in cosmetics.
3^{rd} Group: Substances with anti-oxidant action authorized for use is cosmetics.
   It may happen that part of the retinaldehyde metabolises by oxidation to retinoic acid, due to the action of retinaldehyde dehydrogenase, with the actual retinaldehyde acting as anti-oxidant and that the retinoic acid acts also, as in patent US 20110262373, synergyzing with PPAR-γ which blocks the inflammatory response. However this oxidation and action are produced always inside the keratinocyte.
4^{th} Group: vitamins authorized for use in cosmetics.

For example, the actual retinaldehyde is already the aldehydic form of vitamin A.

The preferred anti-oxidants are Green tea extract, resveratrol and elagic acid.

Also it has been envisaged to include in the formula an activator of PPAR-α, PPAR-β and/or PPAR-γ, other than retinaldehyde, such as for example aloe vera, Green tea, resveratrol, lipoic acid, pyncogenol, L-carnosine, taurine, isoflavones and/or Vitamin E.

The melatonin or N-acetyl-5-methoxytriptamine is a hormone present in superior animals and in some algae, in concentrations that vary according to the daytime/night-time cycle. Melatonin is produced in the pineal gland and synthesized from the neurotransmisor serotonine.

The melatonin in the skin has the function of eliminating the free radicals; it increases the enzymes responsible for metabolising the free radicals and is also capable of oxidising itself to eliminate them without producing a redox cycle, and thus it has been called a suicide anti-oxidant.

The inventors have found that melatonin in the indicated proportions enhances the effect of retinaldehyde in blocking the harmful effects of oxidative stress.

As an advantage, this association of components enhances the action of retinaldehyde, and the composition is not irritant.

### Brief description of the drawings

Other advantages and characteristics of the invention will be appreciated from the following description, wherein, in a non-limiting manner, some preferable embodiments of the invention are described, with reference to the accompanying drawings, wherein:
Fig. 1, evolution of the pores in the skin on the face in a group of people treated with a composition according to the invention.
Fig. 2, evolution of the elasticity of the cheek skin in the group in Fig. 1.
Fig. 3, evolution of the elasticity of the forehead skin in the group in Fig. 1.
Fig. 4, evolution of the bacterial activity in the skin on the face in the group in Fig. 1.
Fig. 5, evolution of the fine wrinkles on the skin on the face in the group in Fig. 1.
Fig. 6, evolution of the hyperpigmentations in the skin on the face in the group in Fig. 1.

### Description of some embodiments of the invention

The inventors formulated and tested a first basic cosmetic composition for rejuvenating the skin ("antiaging") that contained as active ingredients (percentages by weight):
- Retinaldehyde 0.075%
- Melatonin 0.75%
together with suitable excipients.

The results were similar to those obtained with medical compositions (non cosmetic) based on retinoic acid.

A second basic cosmetic composition was formulated and tested, identical to the first, but adding 1% of resveratrol as anti-oxidant:
- Retinaldehyde 0.075%
- Melatonin 0.75%
- Resveratrol 1.00%

The results improved, mainly regarding the pigmentation, apparently enhancing the blocking of the undesirable effects of oxidative stress, since less tissue deterioration was noticed and the dermal structure improved.

A third basic cosmetic composition was formulated and tested, identical to the second, but adding 2% of Green tea as an additional anti-oxidant:
- Retinaldehyde 0.075%
- Melatonin 0.75%
- Resveratrol 1.00%
- Green tea 2.00%

The results improved with respect to the previous basic formulae.

Finally, a fourth basic cosmetic composition was formulated and tested, identical to the first, but adding 5% of aloe vera as an anti-inflammatory:
- Retinaldehyde 0.075%
- Melatonin 0.75%
- Resveratrol 1.00%
- Green tea 2.00%
- Aloe vera 5.00%

Adding aloe vera makes the anti-inflammatory results improve, they are obtained more quickly and toxicity is less.

Probably a factor favouring the synergy is that both the Green tea, and the resveratrol act by improving the activation of the PPAR-γ, and also by favouring that of the actual retinaldehyde.

Clearly a synergistic action is observed between the components indicated as they are added to the basic composition of retinaldehyde and melatonin. The results are being analysed statistically to quantitatively assess the forms individually and the incremental action of each basic formula with respect to the previous one. In every case, the irritation induced is much less than that induced by similar compositions but with retinoic acid.

See the following Table I, summarising the above.

**Table I**

| | | Basic cosmetic composition No. | | | |
|---|---|---|---|---|---|
| | Function | 1 | 2 | 3 | 4 |
| **Retinaldehyde** | Ingredient | 0,08% | 0,075% | 0,08% | 0,075% |
| **Melatonin** | Ingredient | 0.75% | 0.75% | 0.75% | 0.75% |
| **Resveratrol** | Anti-oxidant | - | 1.00% | 1.00% | 1.00% |
| **Green tea** | Anti-oxidant | - | - | 2.00% | 2.00% |
| **Aloe vera** | Anti-inflammatory | - | - | - | 5.00% |

These four basic cosmetic compositions and their possible variants (with additional components) are suitable for use in a method for rejuvenating the skin through the topical application of them.

According to the MASI (MAS1, 2, 3, 4, 5 or 6) skin pigmentation index, six corresponding families of components have been formulated.

The MAS Index (acronym of the expression in English *"Melasma Area and Severity Index*"), is an index used in dermatology and cosmetics to diagnose the severity and the area affected by melasma (or "cloasma"). Melasma is facial hyperpigmentation acquired reoccurringly that is due to the hyperfunction of the melanocytes. The associated factors are internal (UV radiation, infra-red rays, mechanical trauma or friction, psoralens of cosmetics, perfumes, lotions and creams, etc.) and endogenous such as sex, age, colour of skin (phototype III to V), genetic predisposition (cross-breeding), hormonal factors like pregnancy, oral anti-contraceptives, suprarenal and thyroid suffering, etc.). In turn, from each of these six families a specific topical cream has been prepared, that is reflected in the examples.

### Examples

Some preferred compositions are described below, as well as some specific embodiments, that are specific cases of the four basic cosmetic compositions explained above. Each of them is particularly suitable for use in a method for rejuvenating the skin through the topical application of them, preferably in the specific cases of the MASI index indicated in each table.

| MASI 1 (MASI < 10%) | Composition | Example 1 |
|---|---|---|
| Retinaldehyde | 0.05-0.1% | 0,075% |
| Melatonin | 0.5 - 1% | 0.75% |
| Glycyrrhetic acid | 0.5 - 1% | 0.75% |
| Aloe vera | 4 - 6% | 5% |
| Snail slime | 5 - 10% | 7.5% |
| Kojic acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |

| MASI 2 (10 ≤ MASI < 30) | Composition | Example 2 |
|---|---|---|
| Retinaldehyde | 0.05-0.1% | 0,08% |
| Melatonin | 0.5 - 1% | 0.75% |
| Gycyrrhetic acid | 0.5 - 1% | 0.75% |
| Aloe vera | 4 - 6% | 5% |
| Snail slime | 5 - 10% | 7.5% |
| Kojic acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |
| Resveratrol | 0.5 - 1.5% | 1% |

| MASI 3 (30 ≤ MASI < 50) | Composition | Example 3 |
|---|---|---|
| Retinaldehyde | 0.05-0,1% | 0,08% |
| Melatonin | 0.5 - 1% | 0.75% |
| Glycyrrhetic acid | 0,5 - 1% | 0.75% |
| Aloe vera | 4 - 6% | 5% |
| Snail slime | 5 - 10% | 7.5% |
| Kojic Acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |
| Resveratrol | 0.5 - 1.5% | 1% |
| Green tea | 1 - 3% | 2% |

| MASI 4 (50 ≤ MASI < 70) | Composition | Example 4 |
|---|---|---|
| Retinaldehyde | 0.05-0,1% | 0,08% |
| Melatonin | 0.5 - 1% | 0.75% |
| Glycyrrhetic acid | 0.5 - 1% | 0.75% |
| Aloe vera | 4 - 6% | 5% |
| Snail slime | 5 - 10% | 7.5% |
| Kojic acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |
| Resveratrol | 0.5 - 1.5% | 1% |
| Green tea | 1 - 3% | 2% |
| Hyaluronic acid | 0.5 - 1% | 0.75% |

| MASI 5 (70 ≤ MASI < 90) | Composition | Example 5 |
|---|---|---|
| Retinaldehyde | 0.05-0.1% | 0,075% |
| Melatonin | 0,5 - 1% | 0.75% |
| Glycyrrhetic acid | 0,5 - 1% | 0.75% |
| Aloe vera | 4 - 6% | 5% |
| Snail slime | 5 - 10% | 7.5% |
| Kojic acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |
| Resveratrol | 0.5 - 1.5% | 1% |
| Green tea | 1 - 3% | 2% |
| Hyaluronic acid | 0.5 - 1% | 0.75% |
| Glutamine | 0.5 - 1.5% | 1% |
| Grenadine extract | 2 - 4% | 3% |

| MASI 6 (MASI ≥ 90) | Composition | Example 6 |
|---|---|---|
| Retinaldehyde | 0.05-0.1% | 0,075% |
| Melatonin | 0.5 - 1% | 0.75% |
| Glycyrrhetic acid | 0.5 - 1% | 0.75% |
| Aloe vera | 4 - 6% | 5% |
| Snail slime | 5 - 10% | 7.5% |
| Kojic acid | 1 - 2% | 1.5% |
| Stabilised vitamin C | 1.5 - 2.5% | 2% |
| Vitamin E | 0.3 - 0.5% | 0.4% |
| Niacinamide | 1.5 - 2.5% | 2% |
| Resveratrol | 0.5 - 1.5% | 1% |
| Green tea | 1 - 3% | 2% |
| Hyaluronic acid | 0.5 - 1% | 0.75% |
| Glutamine | 0.5 - 1.5% | 1% |
| Grenadine extract | 2 - 4% | 3% |
| N-Acetylglucosamine | 1 - 3% | 2% |

Retinaldehyde is a precursor of retinoic acid, but only produces the oxidative step of aldehyde to acid through the intervention of a cytoplasmatic enzyme. Therefore, to the formula of this invention, which contains the precursor, preferably anti-oxidants will be added not to prevent the step into an acid but to prevent its degradation. The anti-oxidant can be the same as for patent US 20110262373. The step to acid will then be in the skin cells and the formula does not contain anything that accelerates or delays this step.

Retinaldehyde blocks the undesired effects of aging by the reactive oxygen species and synergies with the PPAR-γ that blocks the inflammatory response. The PPAR-γ is activated by the aloe vera, which also behaves as moisturiser or skin regenerator-revitaliser.

Melatonin acts as an eliminator of the free radicals and as an anti-oxidant.

As for the different active ingredients that are added, the depigmentation and anti-oxidant strength increases by combining anti-oxidants (resveratrol+Green tea), with the aloe vera (anti-inflammatory) and the hyaluronic acid (molecule that penetrates the skin acts as "filler" moisturising the deeper layers).

In all the antiaging compositions formulated according to this invention, the irritation induced is much lower than that induced by similar compositions but with retinoic acid, and the therapeutic effects are very similar.

It is important to highlight that within the scope of protection of the invention, it is important to understand that retinaldehyde can be replaced by its technical equivalents. The inventors understand that the function of retinaldehyde is like the retinoic acid precursor, and therefore its technical equivalents are other precursors like for example retinol, retinyl retinoate or retinyl esters.

The precursors of retinoic acid provide a very similar therapeutic effect but without its irritative effects, and, in different degrees, increase stability and reduce photosensitivity.

The compositions of the examples can be completed with variable amounts of other substances, to adapt the formulae to the different degrees of the skin's humidity, and water.

### Application example:

A group of 18 people has used a cosmetic composition according to the invention. Their age was between 30 and 80 years old (30-40 years old: 6 people; 40-50 years old: 4 people; 50-60 years old: 3 people; >60 years old: 5 people) (16 women and 2 men).

The composition was applied to 2 areas of the face: [a] on the forehead, specifically on the cross between the horizontal middle line of the forehead and the vertical middle line between eyebrow arches and the start of the scalp, and [b] on the cheeks, specifically on the cross between the horizontal line of the end of the nasal wing to the ear and the vertical line passing through the middle line of the eye.

The application time was between 2-6 months, on average 3 months.

Formulae with some differences were applied to these people. In general, the formulae used are within the following intervals:

| | |
|---|---|
| Retinaldehyde | 0.05%-0.1% |
| Indometacine | 2%-3% |
| Hydroquinone | 2%-5% |
| Sodium bisulphite | 0.05% |
| Kojic acid | 2-5% |
| Arbutine | 2-4% |
| Acetate tocopherol (vit.E) | 2-5% |
| Vitamin C | 2-5% |
| Melatonine | 0.1% |

It can be considered that the composition applied in these assays is:

| | |
|---|---|
| Retinaldehyde | 0.1% |
| Indometacine | 3% |
| Hydroquinone | 5% |
| Kojic acid | 3% |
| Arbutine | 4% |
| Acetate tocopherol | 3% |
| Vitamin C | 3% |
| Melatonine | 0.1% |

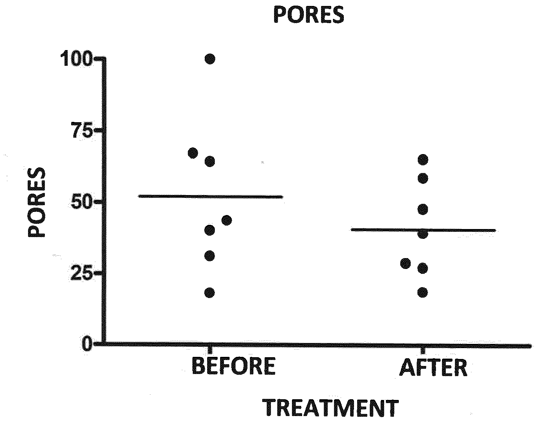

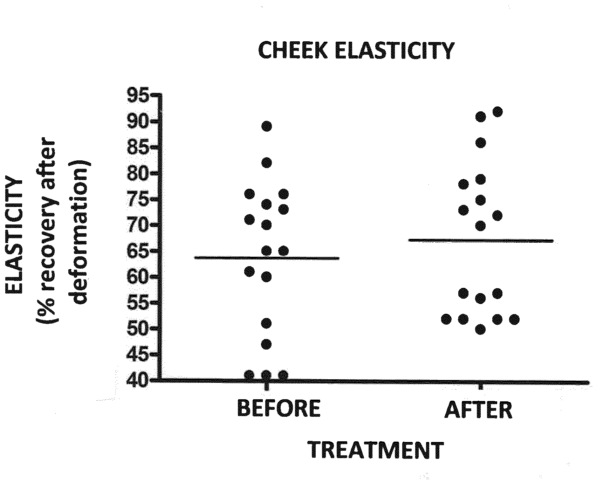

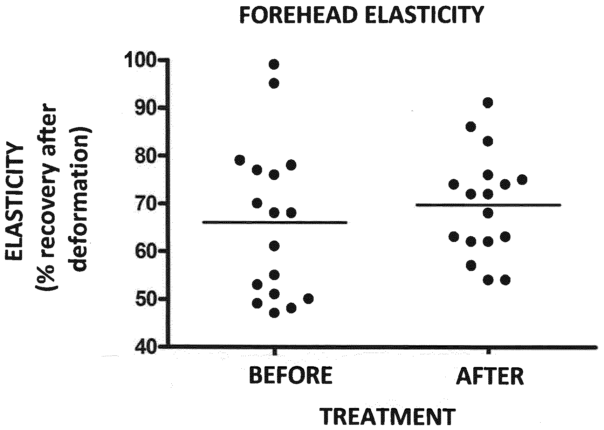

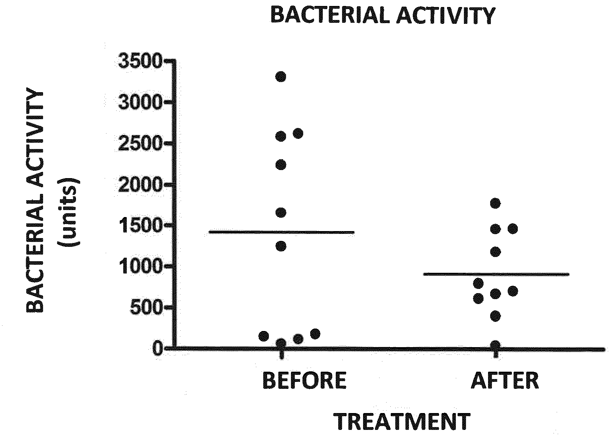

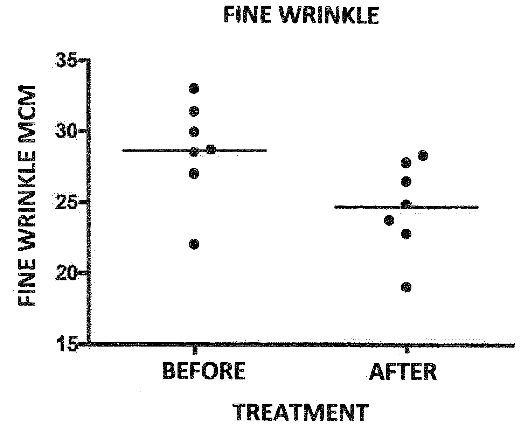

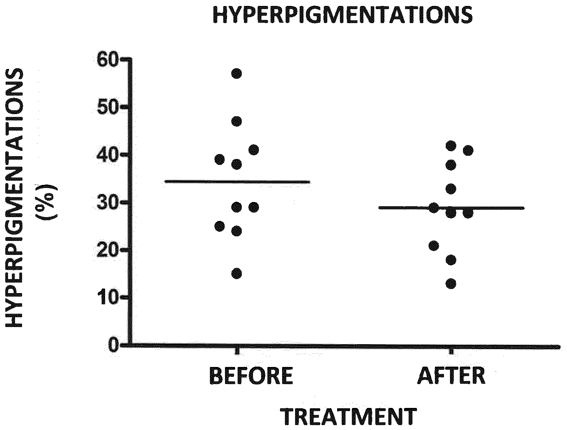

Various parameters have been analysed before and after the treatment. The results are shown in Figs. 1 to 6. A reduction in the pores is observed, and in the bacterial activity, in the fine wrinkles, and in the hyperpigmentations, and an increase in the skin's elasticity both on the cheek and on the forehead.

## Claims

1. Cosmetic composition for rejuvenating the skin **characterized in that** it comprises:
- between 0.01% and 0.5% by weight with respect to the total weight of the composition of a precursor of retinoic acid from the group made up of retinaldehyde, retinol, retinyl retinoate and retinyl esters, and
- between 0.05% and 5% by weight with respect to the total weight of the composition, of melatonin.

2. Cosmetic composition according to claim 1, **characterized in that** it comprises between 0.05% and 0.1% by weight with respect to the total weight of the composition, of retinaldehyde.

3. Cosmetic composition according to any of the claims 1 or 2, **characterized in that** comprises, in addition, at least one anti-inflammatory other than melatonin, preferably from the group made up of liquorice extract, alpha-bisabolol, azulene, glycyrrhetic acid, aloe vera extract and rosmarinum oficinalis extract.

4. Cosmetic composition according to any of the claims 1 a 3, **characterized in that** comprises, in addition, at least one anti-oxidant other than melatonin, preferably from the group made up of Green tea extract, resveratrol and elagic acid.

5. Cosmetic composition according to any of the claims 1 a 4, **characterized in that** it comprises at least one vitamin, preferably from the group made up of vitamin E y vitamin C.

6. Cosmetic composition according to any of the claims 1 to 5, **characterized in that** it comprises an activator of PPAR-α, PPAR-β and PPAR-γ, other than retinaldehyde, preferably from the group made up of: aloe vera, Green tea, resveratrol, lipoic acid, pycnogenol, L-carnosine, taurine, isoflavones and vitamin E.

7. Cosmetic composition according to any of the claims 1 to 6, **characterized in that** it comprises a skin revitalising moisturising element, preferably aloe vera.

8. Cosmetic composition according to one of the preceding claims, **characterized in that** it also comprises hyaluronic acid and/or niacinamide.

9. Cosmetic composition according to one of the preceding claims, **characterized in that** it also comprises one or several depigmentation elements, preferably from the group made up of kojic acid, phytic acid and arbutin.

10. Cosmetic composition according to any of the claims 1 to 9, **characterized in that** it comprises
| | |
|---|---|
| Retinaldehyde | 0.075% |
| Melatonin | 0.75% |

11. Cosmetic composition according to claim 10, **characterized in that** it comprises, additionally
| | |
|---|---|
| Resveratrol | 1.00% |
and/or
| | |
|---|---|
| Green tea | 2.00% |
and/or
| | |
|---|---|
| Aloe vera | 5.00% |

12. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |

13. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |
| Resveratrol | 0.5 - 1.5% |

14. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |
| Resveratrol | 0.5 - 1.5% |
| Green tea | 1 - 3% |

15. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |
| Resveratrol | 0.5 - 1.5% |
| Green tea | 1 - 3% |
| Hyaluronic acid | 0.5 - 1% |

16. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |
| Resveratrol | 0.5 - 1.5% |
| Green tea | 1 - 3% |
| Hyaluronic acid | 0.5 - 1% |
| Glutamina | 0.5 - 1.5% |
| Grenadine extract | 2 - 4% |

17. Cosmetic composition according to any of the claims 1 to 9, **characterized in that** it comprises
| | |
|---|---|
| Retinaldehyde | 0.05-0.1% |
| Melatonin | 0.5 - 1% |
| Glycyrrhetic acid | 0.5 - 1% |
| Aloe vera | 4 - 6% |
| Snail slime | 5 - 10% |
| Kojic acid | 1 - 2% |
| Stabilised vitamin C | 1.5 - 2.5% |
| Vitamin E | 0.3 - 0.5% |
| Niacinamide | 1.5 - 2.5% |
| Resveratrol | 0.5 - 1.5% |
| Green tea | 1 - 3% |
| Hyaluronic acid | 0.5 - 1% |
| Glutamine | 0.5 - 1.5% |
| Grenadine extract | 2 - 4% |
| Acetylglucosamine | 1 - 3% |

## Patentansprüche

1. Kosmetische Zusammensetzung zur Verjüngung der Haut, **dadurch gekennzeichnet, dass** sie umfasst:
- zwischen 0,01 Gew.-% und 0,5 Gew.-%, in Bezug auf das Gesamtgewicht der Zusammensetzung, eines Vorläufers der Retinsäure aus der Gruppe bestehend aus Retinaldehyd, Retinol, Retinylretinoat und Retynilestern, und
- zwischen 0,05 Gew.-% und 5 Gew.-% Melatonin in Bezug auf das Gesamtgewicht der Zusammensetzung.

2. Kosmetische Zusammensetzung nach Anspruch 1, **dadurch gekennzeichnet, dass** sie in Bezug auf das Gesamtgewicht der Zusammensetzung zwischen 0,05 Gew-% und 0,1 Gew.-% Retinaldehyd umfasst.

3. Kosmetische Zusammensetzung nach einem der Ansprüche 1 oder 2, **dadurch gekennzeichnet, dass** sie zusätzlich mindestens einen von Melatonin verschiedenen Entzündungshemmer, vorzugsweise aus der Gruppe bestehend aus Lakritzenextrakt, alpha-Bisabolol, Azulen, Glycyrrhetinsäure, Aloe-Vera-Extrakt und Rosmarinum-oficinalis-Extrakt, umfasst.

4. Kosmetische Zusammensetzung nach einem der Ansprüche 1 bis 3, **dadurch gekennzeichnet, dass** sie zusätzlich mindestens einen von Melatonin verschiedenen Antioxidans, vorzugsweise aus der Gruppe bestehend aus Grüntee-Extrakt, Resveratrol und Ellagsäure, umfasst.

5. Kosmetische Zusammensetzung nach einem der Ansprüche 1 bis 4, **dadurch gekennzeichnet, dass** sie mindestens ein Vitamin, vorzugsweise aus der Gruppe bestehend aus Vitamin E und Vitamin C, umfasst.

6. Kosmetische Zusammensetzung nach einem der Ansprüche 1 bis 5, **dadurch gekennzeichnet, dass** sie einen von Retinaldehyd verschiedenen PPAR-α-, PPAR-β- und PPAP-γ-Aktivator, vorzugsweise aus der Gruppe bestehend aus: Aloe Vera, Grüntee, Resveratrol, Liponsäure, Pycnogenol, L-Carnosin, Taurin, Isoflavonen und Vitamin E, umfasst.

7. Kosmetische Zusammensetzung nach einem der Ansprüche 1 bis 6, **dadurch gekennzeichnet, dass** sie ein hautrevitalisierendes, feuchtigkeitsspendendes Element umfasst.

8. Kosmetische Zusammensetzung nach einem der vorstehenden Ansprüche, **dadurch gekennzeichnet, dass** sie ebenso Hyaluronsäure und/oder Niacinamid umfasst.

9. Kosmetische Zusammensetzung nach einem der vorstehenden Ansprüche, **dadurch gekennzeichnet, dass** sie ebenso ein oder mehrere depigmentierende Elemente, vorzugsweise aus der Gruppe bestehend aus Kojisäure, Phytinsäure und Arbutin, umfasst.

10. Kosmetische Zusammensetzung nach einem der Ansprüche 1 bis 9, **dadurch gekennzeichnet, dass** sie
| | |
|---|---|
| Retinaldehyd | 0,075 % |
| Melatonin | 0,75 % |
umfasst

11. Kosmetische Zusammensetzung nach Anspruch 10, **dadurch gekennzeichnet, dass** sie zusätzlich
| | |
|---|---|
| Resveratrol | 1,00 % |
und/oder
| | |
|---|---|
| Grüntee | 2,00 % |
und/oder
| | |
|---|---|
| Aloe Vera | 5,00 % |
umfasst.

12. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
umfasst.

13. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
| Resveratrol | 0,5 - 1,5 % |
umfasst.

14. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
| Resveratrol | 0,5 - 1,5 % |
| Grüntee | 1 - 3 % |
umfasst.

15. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
| Resveratrol | 0,5 - 1,5 % |
| Grüntee | 1 - 3 % |
| Hyaluronsäure | 0,5 - 1 % |
umfasst.

16. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
| Resveratrol | 0,5 - 1,5 % |
| Grüntee | 1 - 3 % |
| Hyaluronsäure | 0,5 - 1 % |
| Glutamin | 0,5 - 1,5 % |
| Granatapfel-Extrakt | 2 - 4 % |
umfasst.

17. Kosmetische Zusammensetzung nach einem der Ansprüche 1 bis 9, **dadurch gekennzeichnet, dass** sie
| | |
|---|---|
| Retinaldehyd | 0,05 - 0,1 % |
| Melatonin | 0,5 - 1 % |
| Glycyrrhetinsäure | 0,5 - 1 % |
| Aloe Vera | 4 - 6 % |
| Schneckensekret | 5 - 10 % |
| Kojisäure | 1 - 2 % |
| Stabilisiertes Vitamin C | 1,5 - 2,5 % |
| Vitamin E | 0,3 - 0,5 % |
| Niacinamid | 1,5 - 2,5 % |
| Resveratrol | 0,5 - 1,5 % |
| Grüntee | 1 - 3 % |
| Hyaluronsäure | 0,5 - 1 % |
| Glutamin | 0,5 - 1,5 % |
| Granatapfel-Extrakt | 2 - 4 % |
| Acetylglucosamin | 1 - 3 % |
umfasst.

## Revendications

1. Composition cosmétique pour le rajeunissement de la peau **caractérisée en ce qu'**elle comprend :
- entre 0,01 % et 0,5% en poids par rapport au poids total de la composition d'un précurseur d'acide rétinoïque du groupe composé de rétinaldéhyde, rétinol, rétinoate de rétinyle et esters de rétinyle, et
- entre 0,05% et 5% en poids par rapport au poids total de la composition, de mélatonine.

2. Composition cosmétique selon la revendication 1, **caractérisée en ce qu'**elle comprend entre 0,05% et 0,1% en poids par rapport au poids total de la composition, de rétinaldéhyde.

3. Composition cosmétique selon l'une quelconque des revendications 1 ou 2, **caractérisée en ce qu'**elle comprend, en outre, au moins un anti-inflammatoire autre que la mélatonine, de préférence du groupe composé de l'extrait de réglisse, de l'alpha-bisabolol, de l'azulène, de l'acide glycyrrhétique, de l'extrait d'aloe vera et de l'extrait de Rosmarinus officinalis.

4. Composition cosmétique selon l'une quelconque des revendications 1 à 3, **caractérisée en ce qu'**elle comprend, en outre, au moins un antioxydant autre que la mélatonine, de préférence du groupe composé de l'extrait de thé vert, du resvératrol et de l'acide ellagique.

5. Composition cosmétique selon l'une quelconque des revendications 1 à 4, **caractérisée en ce qu'**elle comprend au moins une vitamine, de préférence du groupe composé de la vitamine E et de la vitamine C.

6. Composition cosmétique selon l'une quelconque des revendications 1 à 5, **caractérisée en ce qu'**elle comprend un activateur de PPAR-α, PPAR-β et PPAR-γ, autre que le rétinaldéhyde, de préférence du groupe composé de : l'aloe vera, thé vert, resvératrol, acide lipoïque, pycnogénol, L-carnosine, taurine, isoflavones et vitamine E.

7. Composition cosmétique selon l'une quelconque des revendications 1 à 6, **caractérisée en ce qu'**elle comprend un élément revitalisant hydratant de la peau, de préférence l'aloe vera.

8. Composition cosmétique selon l'une quelconque des revendications précédentes, **caractérisée en ce qu'**elle comprend également de l'acide hyaluronique et/ou de la niacinamide.

9. Composition cosmétique selon l'une quelconque des revendications précédentes, **caractérisée en ce qu'**elle comprend également un ou plusieurs éléments de dépigmentation, de préférence du groupe composé de l'acide kojique, de l'acide phytique et de l'arbutine.

10. Composition cosmétique selon l'une quelconque des revendications 1 à 9, **caractérisée en ce qu'**elle comprend
| | |
|---|---|
| Rétinaldéhyde | 0,075% |
| Mélatonine | 0,75% |

11. Composition cosmétique selon la revendication 10, **caractérisée en ce qu'**elle comprend en outre
| | |
|---|---|
| Resvératrol | 1,00% |
et/ou
| | |
|---|---|
| Thé vert | 2,00% |
et/ou
| | |
|---|---|
| Aloe vera | 5,00% |

12. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |

13. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |
| Resvératrol | 0,5 - 1,5% |

14. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |
| Resvératrol | 0,5 - 1,5% |
| Thé vert | 1 - 3% |

15. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |
| Resvératrol | 0,5 - 1,5% |
| Thé vert | 1 - 3% |
| Acide hyaluronique | 0,5 - 1% |

16. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |
| Resvératrol | 0,5 - 1,5% |
| Thé vert | 1 - 3% |
| Acide hyaluronique | 0,5 - 1% |
| Glutamine | 0,5 - 1,5% |
| Extrait de grenade | 2 - 4% |

17. Composition cosmétique selon l'une quelconque des revendications 1 à 9, **caractérisée en ce qu'**elle comprend
| | |
|---|---|
| Rétinaldéhyde | 0,05 - 0,1% |
| Mélatonine | 0,5 - 1% |
| Acide glycyrrhétique | 0,5 - 1% |
| Aloe vera | 4 - 6% |
| Bave d'escargot | 5 - 10% |
| Acide kojique | 1 - 2% |
| Vitamine C stabilisée | 1,5 - 2,5% |
| Vitamine E | 0,3 - 0,5% |
| Niacinamide | 1,5 - 2,5% |
| Resvératrol | 0,5 - 1,5% |
| Thé vert | 1 - 3% |
| Acide hyaluronique | 0,5 - 1% |
| Glutamine | 0,5 - 1,5% |
| Extrait de grenade | 2 - 4% |
| Acétylglucosamine | 1 - 3% |
